(19) Europäisches Patentamt
European Patent Office
Office européen des brevets

(11) EP 0 918 809 B1

(12) **EUROPÄISCHE PATENTSCHRIFT**

(45) Veröffentlichungstag und Bekanntmachung des Hinweises auf die Patenterteilung:
**07.11.2001 Patentblatt 2001/45**

(21) Anmeldenummer: **97938925.1**

(22) Anmeldetag: **18.08.1997**

(51) Int Cl.[7]: **C08G 18/78**, C08G 18/08, C08G 18/32, C08G 18/30, C07C 275/62

(86) Internationale Anmeldenummer:
**PCT/EP97/04505**

(87) Internationale Veröffentlichungsnummer:
**WO 98/07771 (26.02.1998 Gazette 1998/08)**

(54) **VERFAHREN ZUR HERSTELLUNG VON BIURETGRUPPEN ENTHALTENDEN POLYISOCYANATEN AUS (CYCLO)ALIPHATISCHEN DIISOCYANATEN**

PROCESS FOR PRODUCING POLYISOCYANATES CONTAINING BIURET GROUPS FROM (CYCLO) ALIPHATIC DIISOCYANATES

PROCEDE POUR LA PREPARATION DE POLYISOCYANATES, CONTENANT DES GROUPES BIURET, A PARTIR DE DIISOCYANATES (CYCLO) ALIPHATIQUES

(84) Benannte Vertragsstaaten:
**BE DE ES FR GB IT NL SE**

(30) Priorität: **19.08.1996 DE 19633404**

(43) Veröffentlichungstag der Anmeldung:
**02.06.1999 Patentblatt 1999/22**

(73) Patentinhaber: **BASF AKTIENGESELLSCHAFT**
**67056 Ludwigshafen (DE)**

(72) Erfinder:
- **BRUCHMANN, Bernd**
**D-67251 Freinsheim (DE)**
- **REIF, Martin**
**D-67063 Ludwigshafen (DE)**
- **HOFSCHEUER, Werner**
**D-67141 Neuhofen (DE)**
- **JÄHME, Joachim**
**D-67240 Bobenheim-Roxheim (DE)**
- **LANGER, Werner**
**D-67061 Ludwigshafen (DE)**
- **RENZ, Hans**
**D-67149 Meckenheim (DE)**
- **MOHRHARDT, Günter**
**D-67346 Speyer (DE)**
- **SCHIESSL, Michael**
**D-67454 Hassloch (DE)**

(74) Vertreter: **Isenbruck, Günter et al**
**Patent- und Rechtsanwälte**
**Bardehle et al.**
**Theodor-Heuss-Anlage 12**
**68165 Mannheim (DE)**

(56) Entgegenhaltungen:
**EP-A- 0 277 353**
**DE-A- 1 543 178**
**DE-B- 2 609 995**
**US-A- 3 903 126**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 918 809 B1

**Beschreibung**

Die Erfindung betrifft ein Verfahren zur Herstellung von Biuretgruppen enthaltenden Polyisocyanaten aus (cyclo)aliphatischen Diisocyanaten und mindestens einem Amin oder Wasser oder einem Gemisch davon, ggf. zusammen mit mindestens einem Alkohol, als Reaktanden, die in einem Mischelement mit hoher Scherwirkung miteinander vermischt werden. Biuretgruppen aufweisende (cyclo)aliphatische Polyisocyanate werden u.a. in hochwertigen licht- und wetterbeständigen Zweikomponenten-PUR-Lacken, in Klebstoffen und Dispersionen eingesetzt. Zur Biuretherstellung werden die Diisocyanate mit einer definierten Menge eines Biuretisierungsmittels, z.B. Wasser, wasserabspaltenden Substanzen, Aminen oder Harnstoffen, versetzt und üblicherweise bei Temperaturen zwischen 100 und 200 °C umgesetzt. Anschließend wird das überschüssige Isocyanat-Monomer durch ein- oder mehrstufige Destillation abgetrennt. Eine gute Übersicht über die verschiedenen Herstellungsmöglichkeiten von Biureten geben u.a. die DE-A 34 03 277, die EP-A 0 716 080, die DE-A 195 25 474 sowie ein Übersichtsartikel in J. prakt. Chem. 336 (1994), S. 185-200.

Aus der Literatur sind Verfahren zur Direktherstellung von Biureten aus Isocyanaten und Aminen bereits bekannt.

So beschreibt beispielsweise die DE-A 22 61 065 u.a. (Beispiel 16) die Umsetzung von überschüssigen Mengen an 1,6-Hexamethylendiisocyanat (HDI) mit 1,6-Hexamethylendiamin (HDA). Gemäß dieses Beispiels werden die Reaktionspartner 12 Stunden lang bei 180 °C gerührt. Dieses lange Nachheizen bei hoher Temperatur ist nicht nur in höchstem Maße unwirtschaftlich, sondern führt insbesondere unter großtechnischen Produktionsbedingungen zu einer Verfärbung des Reaktionsprodukts, so daß dessen Verwendung in lichtechten Lacken enge Grenzen gesetzt sind. Die Nacharbeitung des oben genannten Beispiels 16 ergab ein hochviskoses Biuret mit einem erheblichen Anteil an Feststoff. Es ist also nicht möglich, gemäß des dort beschriebenen Verfahrens ein von monomerem Ausgangsdiisocyanat freies Biuretpolyisocyanat zu erhalten, welches völlig frei von unlöslichen, gelartigen Nebenprodukten ist.

Die DE-A 26 09 995 versucht die Nachteile der Feststoffbildung gemäß der DE-A 22 61 065 zu umgehen, indem die Amine gasförmig bei Temperaturen von 100 bis 250 °C in das vorgelegte Diisocyanat eingebracht werden. Durch die stets starke Verdünnung der gasförmig eingeleiteten Diamine treten Polyharnstoff-Ausfällungen in wesentlich geringerem Maße auf, jedoch kann auch dort die Bildung von Harnstoffagglomeraten nicht vollständig vermieden werden, da sich diese durch lokale Überkonzentration der Reaktanden an der Düse selbst bilden und damit diese verstopfen. Ferner benötigt man für die Ausführung dieses Verfahren in technischem Maßstab, bedingt durch die Verwendung von gasförmigen Diaminen, große Volumina, was die Kontrolle der Reaktionsbedingungen schwierig macht.

Die EP-B 0 003 505 versucht das Problem der Verstopfung von Düsen zu umgehen, indem das Amin mittels einer Glattstrahldüse unter hohem Druck ($2 \cdot 10^5$ bis $1 \cdot 10^8$ Pa) bei Temperaturen von -20 bis 250 °C in das vorgelegte Isocyanat eingedüst wird.

In dem in der EP-B 0 277 353 beschriebenen Verfahren werden zur Vermeidung der Feststoffbildung die Amine, ggf. unter Zusatz von Wasser oder Alkoholen, bei Temperaturen oberhalb 250 °C unter Drücken von bis zu $1 \cdot 10^7$ Pa mittels Düsen in einer Mischkammer mit dem Isocyanat vereinigt. Die Nachreaktion zur Einstellung der Molekulargewichtsverteilung erfolgt anschließend in einem Rührkessel bei Temperaturen von 80 bis 220 °C. Ein solches Verfahren erfordert aufgrund der hohen Drücke und der Düsentechnologie einen großen technologischen Aufwand. Die Feststoffbildung kann in den nachgeschalteten Reaktoren jedoch immer noch ein Problem darstellen. Die hohen Temperaturen von oberhalb 250 °C reichen bereits an die Zersetzungstemperatur von z.B. HDI heran, so daß eine thermische Schädigung des Produktes, die sich in einer dunklen Färbung manifestiert, nicht ausgeschlossen werden kann.

Wie sich aus obigem ergibt, ist es mit den bislang beschriebenen Verfahren nur ausgesprochen schwierig möglich, nahezu farblose Biuretgruppen enthaltende Polyisocyanate herzustellen, ohne daß dabei gleichzeitig hohe Mengen an störenden Feststoffen gebildet werden. Da Biuretgruppen enthaltende Polyisocyanate bevorzugt im Klarlack-Sektor eingesetzt werden, sind Verfärbungen sowie ein hoher Feststoffgehalt dieser Isocyanate nachteilig. Viele der oben beschriebenen Verfahren können darüber hinaus nur mit einem hohen technologischen Aufwand unter großtechnischen Produktionsbedingungen betrieben werden.

Der vorliegenden Erfindung lag demnach die Aufgabe zugrunde, ein Verfahren zur Herstellung von Biuretgruppen enthaltenden Polyisocyanaten zur Verfügung zu stellen, das frei von den oben beschriebenen Nachteilen ist.

Überraschenderweise wurde nunmehr gefunden, daß sich Biuretgruppen enthaltende Polyisocyanate mit marktüblichen Viskositäten zwischen 3.000 und 10.000 mPas und guten Farbzahlen von 500 Hz oder weniger, vorzugsweise 300 Hz oder weniger, insbesondere 100 oder weniger, auch bei moderaten Bedingungen erzeugen lassen, indem man ein aliphatisches, cycloaliphatisches oder araliphatisches Diisocyanat und mindestens ein Amin oder Wasser oder ein Gemisch aus Wasser und mindestens einem Amin und ggf. einen Alkohol vermischt und in einem Mischelement mit hoher Scherwirkung miteinander in Kontakt bringt.

Demgemäß betrifft die vorliegende Erfindung ein Verfahren zur Herstellung eines Biuretgruppen enthaltenden Polyisocyanats aus mindestens einem aliphatischen, cycloaliphatischen oder araliphatischen Diisocyanat oder einem

Gemisch aus zwei oder mehr davon und mindestens einem Amin oder Wasser oder einem Gemisch aus Wasser und mindestens einem Amin als Reaktanden, wobei die Reaktanden in einem Mischelement mit hoher Scherwirkung, die durch Schergeschwindigkeiten in einem Bereich von 100 bis 200.000 $s^{-1}$ hervorgerufen wird, miteinander vermischt werden.

Die im Rahmen der vorliegenden Erfindung verwendete Bezeichnung "Biuretgruppen enthaltendes Polyisocyanat" betrifft Polyisocyanate, wie im Rahmen der vorliegenden Erfindung definiert, die als Hauptkomponente Moleküle der allgemeinen Formel

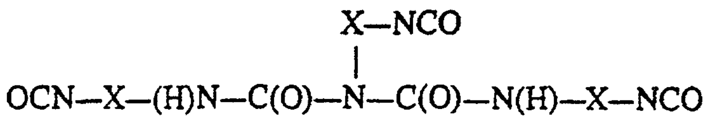

sowie deren homologe Isomeren enthalten, wobei X für eine aliphatische, cycloaliphatische oder araliphatische Alkylengruppe steht.

Als Ausgangsprodukte für die Herstellung der Biuretgruppen enthaltenden Polyisocyanate kommen die an sich bekannten aliphatischen, cycloaliphatischen oder araliphatischen Diisocyanate allein oder in Gemischen von zwei oder mehr davon in Betracht. Vorzugsweise sind dies Alkylendiisocyanate mit 4 bis 12 Kohlenstoffatomen im Alkylenrest, wie z.B. 1,4-Tetramethylendiisocyanat, 1,6-Hexamethylendiisocyanat (HDI), 1,12-Dodecamethylendiisocyanat, 2-Ethyl-tetramethylen-diisocyanat-1,4, 2-Methyl-pentamethylen-diisocyanat-1,5, 2,2,4-Trimethyl-hexamethylendiisocyanat-1,6, 2,4,4-Trimethylhexamethylendiisocyanat-1,6, Lysinalkylesterdiisocyanate; cycloaliphatische Diisocyanate, wie z.B. Cyclohexan-1,3- und -1,4-diisocyanat, Isophorondiisocyanat (IPDI) und Bis(4-isocyanatocyclohexyl)methan, 2,5- und 2,6-Diisocyanatomethylnorbornan, oder araliphatische Diisocyanate wie Xylylendiisocyanat und Tetramethylxylylendiisocyanat, wobei besonders bevorzugt 2-Butyl-2-ethylpentamethylen-diisocyanat, 2-Methylpentamethylen-diisocyanat, IPDI, HDI und Bis(4-isocyanatocyclohexyl)methan eingesetzt werden.

Weitere Ausgangsmaterialien für das erfindungsgemäße Verfahren sind organische Mono- und Diamine mit aliphatisch und/oder cycloaliphatisch gebundenen primären oder sekundären Aminogruppen. Dabei sind beispielsweise aliphatische oder cycloaliphatische Monoamine der Formel $R\text{-}NH_2$, in welcher R für einen aliphatischen Kohlenwasserstoffrest mit 1 bis 12 Kohlenstoffatomen oder einem cycloaliphatischen Kohlenwasserstoffrest mit 5 bis 7 Kohlenstoffatomen steht, wie z.B. Methylamin, n-Butylamin, n-Dodecylamin, Cyclopentylamin, Cyclohexylamin oder Cycloheptylamin zu nennen. Weiterhin kann auch Ammoniak in die Betrachtungen einbezogen werden.

Ferner sind primäre Aminogruppen aufweisende Diamine der Formel $R'(NH_2)_2$, in welcher R' für einen aliphatischen Kohlenwasserstoffrest mit 2 bis 12 Kohlenstoffatomen oder einen cycloaliphatischen Kohlenwasserstoffrest mit 4 bis 17 Kohlenstoffatomen steht. Beispielhaft zu nennen sind hier Ethylendiamin, 1,2- und 1,3-Propylendiamin, 1,4-Diaminobutan, 2,2-Dimethylpropandiamin-(1,3), 1,6-Hexamethylendiamin, 2,5-Dimethylhexandiamin-(2,5), 2,2,4-Trimethylhexandiamin-(1,6), 1,8-Diaminoctan, 1,10-Diaminodecan, 1,11-Undecandiamin, 1,12-Dodecandiamin, 1-Methyl-4-aminoisopropyl-cyclohexylamin-1, 3-Aminomethyl-3,5,5-trimethylcyclohexylamin-(1), 1,2-Bis-(aminomethyl)-cyclobutan, 1,2- und 1,4-Diaminocyclohexan, 1,2- und 1,4-, 1,5- und 1,8-Diaminodecalin, 1-Methyl-4-aminoisopropyl-cyclohexylamin-1, 4,4'-Diamino-dicyclohexyl, Bis(4-amino-cyclohexyl)methan, 2,2'-Bis(4-aminocyclohexyl)-propan, 3,3'-Dimethyl-4,4'-diamino-dicyclohexylmethan, 1,2-Bis-(4-aminocyclohexyl)-ethan, 3,3'-5,5'-Tetramethyl-bis-(4-aminocyclohexyl)-methan und -propan und Isophorondiamin sowie Gemische ans zwei oder mehr davon.

Besonders bevorzugt werden als Amine Hexamethylendiamin, Isophorondiamin sowie Bis(4-aminocyclohexyl)-methan eingesetzt.

In einer besonders bevorzugten Ausführungsform der vorliegenden Erfindung werden als Diisocyanat Hexamethylendiisocyanat und als Amin Hexamethylendiamin eingesetzt.

Beim erfindungsgemäßen Verfahren können die Amine auch in Abmischung mit Wasser und/oder aliphatischen Alkoholen eingesetzt werden, wobei insbesondere tertiäre Alkohole eingesetzt werden. Beispielhaft zu nennen sind 1,4-Dihydroxybutan, Neopentylglykol, Trimethylolpropan, Glycerin, tert.-Butanol, wobei tert.-Butanol besonders bevorzugt ist. Ebenso kann Wasser allein eingesetzt werden.

Bei der Durchführung des erfindungsgemäßen Verfahrens werden die Ausgangsdiisocyanate und die Amine, Wasser sowie deren Gemische, ggf. mit einem Alkohol, in einem solchen Mengenverhältnis zur Umsetzung gebracht, daß das Äquivalentverhältnis von Isocyanatgruppen zu den mit Isocyanatgruppen reaktiven Gruppen von mindestens 2:1, vorzugsweise von 4:1 bis 25:1 und insbesondere von 7:1 bis 20:1, entsprechen, wobei die primären Aminogruppen als difunktionelle Gruppen in die Berechnung eingehen. Dabei werden die Reaktanden vorzugsweise kontinuierlich in dem oben erwähnten Mischungsverhältnis in den Reaktor eingebracht.

Erfindungswesentlich ist, daß die im Rahmen des vorliegenden Verfahrens verwendeten Reaktanden in einem

Mischelement mit hoher Scherwirkung miteinander in Kontakt gebracht werden.

Der Begriff "hohe Scherwirkung" steht hier für Scherkräfte, die durch Schergeschwindigkeiten in einem Bereich von ungefähr 100 bis ungefähr 200.000 $s^{-1}$, vorzugsweise ungefähr 1.000 bis ungefähr 100.000 $s^{-1}$, und insbesondere ungefähr 3.000 bis ungefähr 30.000 $s^{-1}$, hervorgerufen werden.

Die Schergeschwindigkeit γ wird dabei folgendermaßen definiert:

$$\dot{\gamma} = \frac{r \cdot \Omega}{b}$$

r = Radius des Rotors (mm); Ω = Winkelgeschwindigkeit ($s^{-1}$); b = Spaltbreite zwischen Rotor und Stator (mm).

Zur Erzeugung der oben beschriebenen Scherkräfte bzw. des Scherfeldes können prinzipiell alle dazu geeigneten Mischelemente verwendet werden. Beispielhaft zu nennen sind Suspendier- oder Dispergierwerkzeuge bestehend aus Rotor/Stator- und ggf. Förderelementen, wobei insbesondere ULTRA-TURRAX-Dispergiervorrichtungen, Intensivmischer, Scherscheibenmischer, Extruder, Mühlen, usw. zu nennen sind. Weiterhin sind auch Ultraschall-Mischvorrichtungen zu nennen.

Im Rahmen des erfindungsgemäßen Verfahrens werden die Reaktanden bei Temperaturen von ungefähr 20 bis ungefähr 280 °C, vorzugsweise ungefähr 80 bis ungefähr 250 °C und insbesondere ungefähr 100 bis ungefähr 220 °C, in einem wie oben dargestellt erzeugten Scherfeld zusammengegeben und anschließend in an sich bekannter Weise umgesetzt.

Dabei wird in der Regel das Diisocyanat vorgelegt, das entsprechende Scherfeld eingestellt und der Reaktor auf eine geeignete Temperatur aufgeheizt. Anschließend wird Wasser, das ggf. erwärmte Mono- oder Diamin oder das Gemisch aus zwei oder mehr davon, ggf. mit einem Alkohol vermischt, zugegeben, wobei ebenfalls ein Katalysator vorhanden sein kann, und vermischt.

Nach dem Vermischen bzw. dem Durchlaufen der Vermischkammer und der dieser ggf. nachgeschalteten Verweilzeitstrecke wird das Reaktionsgemisch anschließend, vorzugsweise in einem Zeitraum von weniger als ungefähr 20 Minuten, weiter bevorzugt in einem Zeitraum von ungefähr 10 Minuten, stetig oder stufenweise auf Reaktionstemperatur gebracht, die im allgemeinen zwischen ungefähr 80 bis ungefähr 250 °C, vorzugsweise bei ungefähr 100 °C bis ungefähr 220 °C, liegt, und bei dieser Temperatur über einen Zeitraum von höchstens ungefähr 5 Stunden, vorzugsweise höchstens ungefähr 2 Stunden, einer thermischen Nachbehandlung unterzogen.

Die thermische Nachbehandlung kann beispielsweise in einem Reaktor, in kaskadenförmig angeordneten Reaktoren, in kontinuierlich durchflossenen Rührkesseln oder in einem Rohrreaktor durchgeführt werden.

Im Anschluß an die thermische Nachbehandlung liegt als Reaktionsprodukt eine Lösung von Biuretgruppen aufweisenden Polyisocyanaten in überschüssigem Ausgangsdiisocyanat vor, die unmittelbar nach der thermischen Nachbehandlung oder zu einem späteren Zeitpunkt destillativ oder auch durch Extraktion mit beispielsweise n-Hexan von überschüssigem Ausgangsdiisocyanat befreit werden kann.

Vorzugsweise wird das gesamte oben beschriebene Herstellungsverfahren kontinuierlich betrieben.

Auf diese Weise sind hochwertige Polyisocyanate mit Biuretstruktur erhältlich, die einen Gehalt an überschüssigem Ausgangsdiisocyanat von maximal 0,5 Gew.-% aufweisen.

Die erfindungsgemäß hergestellten Biuretgruppen enthaltenden Polyisocyanate zeichnen sich durch gute Farbzahlen, eine gute Lagerstabilität und eine gute Verdünnbarkeit mit aprotischen Lösungsmitteln aus.

Sie können als Vernetzerkomponente in licht- und wetterbeständigen Zweikomponenten-PUR-Lacken sowie bei der Herstellung von PUR-Weich- und Hartschaumstoffen, in Klebstoffen und (Anstrich)dispersionen verwendet werden.

Im folgenden soll die vorliegende Erfindung anhand von einigen Beispielen erläutert werden.

**BEISPIELE**

Beispiel 1

In einem 2 1-Rührreaktor wurde unter Stickstoffbedeckung Hexamethylendiisocyanat (HDI) (1000 g) vorgelegt, der Ultra-Turrax eingetaucht, auf 9000 U/min eingestellt und das Isocyanat auf 80 °C aufgeheizt. Innerhalb von 10 min wurde auf 80 °C erwärmtes Hexamethylendiamin (HDA) zugegeben.

Anschließend wurde auf die in Tabelle 1 angegebene Reaktionstemperatur aufgeheizt und während der angegebenen Reaktionsdauer mit dem Turrax weitergerührt. Nach dem Abkühlen wurde die Reaktionslösung zum Entfernen des nicht umgesetzten HDI am Dünnschichtverdampfer bei 165 °C Öltemperatur und einem Druck von $2{,}5 \cdot 10^2$ Pa destilliert.

**Tabelle 1**

**Reaktionen von HDI mit HDA**

| HDA [g] | Reaktions-temperatur [°C] | Reaktions-zeit [min] | Visk. 25 °C [mPas] | NCO [Gew.-%] |
|---|---|---|---|---|
| 30 | 180 | 180 | 3250 | 22,4 |
| 40 | 210 | 30 | 4650 | 22,2 |
| 40 | 230 | 15 | 12000 | 19,9 |

Beispiel 2

In einem 2 1-Rührreaktor wurde unter Stickstoffbedeckung HDI (1000 g) vorgelegt, der Ultra-Turrax eingetaucht, auf 9000 U/min eingestellt und das Isocyanat auf 80 °C aufgeheizt. Innerhalb von 10 min wurde auf 80 °C erwärmtes Hexamethylendiamin, Wasser, deren Gemische oder die tert.-Butanol-/HDA-Mischung und ggf. Katalysator zugegeben (siehe Tabellen 2 und 3).

Anschließend wurde der Turrax entfernt und durch einen konventionellen Ankerrührer ersetzt. Die Reaktionsmischung wurde danach auf die in den Tabellen 2 und 3 angegebenen Reaktionstemperaturen aufgeheizt und während der angegebenen Reaktionsdauer mit dem Ankerrührer weitergerührt. Nach dem Abkühlen wurde das Reaktionsgemisch zum Entfernen des nicht umgesetzten HDI am Dünnschichtverdampfer bei 165 °C Öltemperatur und einem Druck von $2{,}5 \cdot 10^2$ Pa destilliert.

**Tabelle 2**

**Reaktionen von HDI mit HDA, Wasser oder HDA/Wasser-Gemischen**

| HDA [g] | Wasser [g] | Katalysator [mol-% bez. auf HDI] | Reaktionstemperatur [°C] | Reaktionszeit [min] | Visk. 25 °C [mPas] | NCO [Gew.-%] |
|---|---|---|---|---|---|---|
| 30 | - | - | 180 | 300 | 5310 | 21,9 |
| 20 | - | - | 190 | 300 | 4650 | 22,0 |
| - | 10 | DBP (0,2) | 150 | 60 | 3220 | 22,6 |
| 7 | 7 | DEHP (1,0) | 210 | 60 | 6430 | 21,2 |
| 15 | 5 | DEHP (1,0) | 210 | 60 | 8410 | 20,9 |

**Tabelle 3**

**Reaktion von HDI mit HDA/tert.-Butanol-Mischung**

| HDA [g] | tert.-Butanol [g] | Katalysator [mol-% bez. auf HDI] | Reaktionstemperatur [°C] | Reaktionszeit [min] | Visk. 25 °C [mPas] | NCO [Gew.-%] |
|---|---|---|---|---|---|---|
| 7 | 27 | DEHP (1,0) | 180 | 300 | 3480 | 22,6 |

**DBP = Dibutylphosphat**
**DEHP = Di(2-ethylhexyl)phosphat**

Beispiel 3

In einen Intensivmischer 1, bestehend aus vier separat beheizbaren, hintereinandergeschalteten Mischelementen 1a - 1d (siehe Fig. 1) wurde bei einer Rotorgeschwindigkeit von 4000 U/min kontinuierlich auf 80 °C erwärmtes HDI 2 gegeben. Im zweiten Mischelement 1b wurde auf 80 °C erwärmtes HDA, Wasser oder das Gemisch aus HDA und Wasser 3 eindosiert. Die Verweilzeit der Reaktionsmischung kann durch den Volumenstrom beeinflußt werden, sie betrug etwa 0,5 - 2 min. Anschließend gelangte das Reaktionsgemisch in einen beheizten Rührreaktor 4, in dem die Reaktion vervollständigt wurde. Die Verweilzeit betrug hier 0,5 - 2 h. Zur Abtrennung des nicht umgesetzten, monomeren HDI erfolgte eine Destillation im Dünnschichtverdampfer bei einer Öltemperatur von 165 °C und einem Druck von $2{,}5 \cdot 10^2$ Pa. Die Reaktionsdaten sind in Tabelle 4 zusammengestellt.

**Tabelle 4**

**Daten der Intensivmischer-Versuche**

| HDI [g/h] | HDA [g/h] | Wasser [g/h] | Temperatur I-Mischer [°C] | Temperatur Kessel [°C] | NCO Biuret [%] | Visk. 25 °C [mPas] |
|---|---|---|---|---|---|---|
| 1000 | 20 | - | 180 | 190 | 21,9 | 4250 |
| 2000 | 40 | - | 180 | 210 | 21,8 | 5230 |
| 2000 | - | 20 | 150 | 150 | 22,2 | 4350 |
| 1000 | 7 | 7 | 180 | 190 | 22,0 | 4130 |

Vergleichsbeispiel 1 (nach DE-OS 22 61 065)

In einem 2 1-Rührrekator wurde unter Stickstoffbedeckung HDI (1000 g) bei 25 °C vorgelegt und unter schnellem Rühren mittels Ankerrührer innerhalb von 25 min 86.3 g auf 70 °C erwärmtes Hexamethylendiamin zugegeben. Sofort nach der Zugabe fiel polymerer Harnstoff als Feststoff aus. Die Reaktionsmischung wurde auf 180 °C erwärmt und 12 h bei dieser Temperatur gerührt. Die Mischung war dunkel gefärbt und enthielt noch erhebliche Mengen an Feststoff. Nach der Filtration wurde ein gelbbraun gefärbtes Biuret mit einer Viskosität > 25000 mPas (25 °C) erhalten, was nicht im Dünnschichtverdampfer zur Entfernung von monomerem HDI destilliert werden konnte.

Vergleichsbeispiel 2

(mit verringertem HDA-Anteil, analog zu den erfindungsgemäßen Beispielen in Tabelle 2, jedoch ohne Verwendung eines Mischelements mit hoher Scherwirkung)

In einem 2 1-Rührreaktor wurde unter Stickstoffbedeckung HDI (1000 g) bei 80 °C vorgelegt und unter schnellem Rühren mittels Ankerrührer innerhalb von 10 min 20 g auf 80 °C erwärmtes Hexamethylendiamin zugegeben. Sofort nach der Zugabe fiel polymerer Harnstoff als Feststoff aus. Die Reaktionsmischung wurde auf 190 °C erwärmt und 5 h bei dieser Temperatur gerührt. Die Mischung war gelb gefärbt und enthielt noch erhebliche Mengen an Feststoff. Nach der Filtration und Destillation wurde in 10% Ausbeute ein Biuret mit einer Viskosität von 1190 mPas (25 °C) und einem NCO-Gehalt von 24,1 Gew.-% erhalten.

**Patentansprüche**

1. Verfahren zur Herstellung eines Biuretgruppen enthaltenden Polyisocyanates aus mindestens einem aliphatischen oder cycloaliphatischen oder araliphatischen Diisocyanats oder einem Gemisch aus zwei oder mehr davon und mindestens einem Amin oder Wasser oder einem Gemisch aus Wasser und mindestens einem Amin als Reaktanden, wobei die Reaktanden in einem Mischelement mit hoher Scherwirkung, die von Schergeschwindigkeiten im Bereich von 100 bis 200.000 $s^{-1}$ hervorgerufen wird, miteinander vermischt und zur Reaktion gebracht werden.

2. Verfahren nach Anspruch 1, **dadurch gekennzeichnet, daß** das aliphatische, cycloaliphatische oder araliphatische Diisocyanat ein Alkylendiisocyanat mit 4 bis 12 Kohlenstoffatomen im Alkylenrest oder ein Gemisch aus zwei oder mehr davon ist.

3. Verfahren nach Anspruch 1 oder 2, **dadurch gekennzeichnet, daß** das mindestens eine Amin, Wasser oder das Gemisch aus Wasser und mindestens einem Amin zusätzlich mit mindestens einem Alkohol vermischt wird.

4. Verfahren nach Anspruch 3, **dadurch gekennzeichnet, daß** der mindestens eine Alkohol ein tertiärer Alkohol ist.

5. Verfahren nach einem der vorhergehenden Ansprüche, **dadurch gekennzeichnet, daß** das mindestens eine Amin Hexamethylendiamin, Isophorondiamin oder Bis(4-aminocyclohexyl)methan ist.

6. Verfahren nach einem der vorhergehenden Ansprüche, **dadurch gekennzeichnet, daß** als Diisocyanat Hexamethylendiisocyanat und als das mindestens eine Amin Hexamethylendiamin eingesetzt werden.

7. Verfahren nach einem der vorhergehenden Ansprüche, **dadurch gekennzeichnet, daß** es kontinuierlich betrieben wird.

**Claims**

1. A process for preparing a polyisocyanate containing biuret groups from at least one aliphatic, cycloaliphatic or araliphatic diisocyanate or a mixture of two or more thereof and at least one amine and or water or a mixture of water and at least one amine as reactants, the reactants being mixed with one another and reacted in a mixing unit having a high shear action which is induced by shear rates in the range of from 100 to 200,000 $s^{-1}$.

2. A process as claimed in claim 1, wherein the aliphatic, cycloaliphatic or araliphatic diisocyanate is an alkylene diisocyanate having 4 to 12 carbons in the alkylene radical or is a mixture of two or more thereof.

3. A process as claimed in claim 1 or 2, wherein the at least one amine, water or the mixture of water and at least one amine is additionally mixed with at least one alcohol.

4. A process as claimed in claim 3, wherein the at least one alcohol is a tertiary alcohol.

5. A process as claimed in any of the preceding claims, wherein the at least one amine is hexamethylenediamine, isophoronediamine or bis(4-aminocyclohexyl)methane.

6. A process as claimed in any of the preceding claims, wherein the diisocyanate employed is hexamethylene diisocyanate and the at least one amine employed is hexamethylenediamine.

7. A process as claimed in any of the preceding claims, which is operated continuously.

**Revendications**

1. Procédé pour la préparation d'un polyisocyanate, contenant des groupes biuret à partir d'au minimum un diisocyanate aliphatique ou cyclo-aliphatique ou araliphatique ou d'un mélange de deux ou plusieurs de ceux-ci et au minimum d'une amine ou de l'eau, ou un mélange d'eau et au minimum d'une amine, en tant que réactifs, en mélangeant et en faisant réagir les réactifs les uns avec les autres dans un mélangeur avec un effet de cisaillement élevé, provoqué par des vitesses de cisaillement de 100 à 200 000 $s^{-1}$.

2. Procédé selon la revendication 1, **caractérisé en ce que** le diisocyanate aliphatique, cyclo-aliphatique ou araliphatique est un diisocyanate d'alkylène avec 4 à 12 atomes de carbone dans le résidu alkylène ou un mélange de deux ou plusieurs de ceux-ci.

3. Procédé selon la revendication 1 ou 2, **caractérisé en ce que** l'on mélange l'amine ou les amines en question, l'eau ou le mélange d'eau et au minimum d'une amine, avec en plus au minimum un alcool.

4. Procédé selon la revendication 3, **caractérisé en ce que** l'alcool, ou les alcools en question, est un alcool tertiaire.

5. Procédé selon l'une des quelconques revendications précédentes, **caractérisé en ce que** l'amine, ou les amines en question, est l'hexaméthylènediamine, l'isophoronediamine ou le bis (4-aminocyclohexyl)méthane.

6. Procédé selon l'une quelconque des revendications précédentes, **caractérisé en ce que** l'on utilise le diisocyanate d'hexaméthylène en tant que diisocyanate et l'hexaméthylènediamine en tant qu'amine ou amines en question.

**7.** Procédé selon l'une quelconque des revendications, **caractérisé en ce qu'**on le réalise en continu.

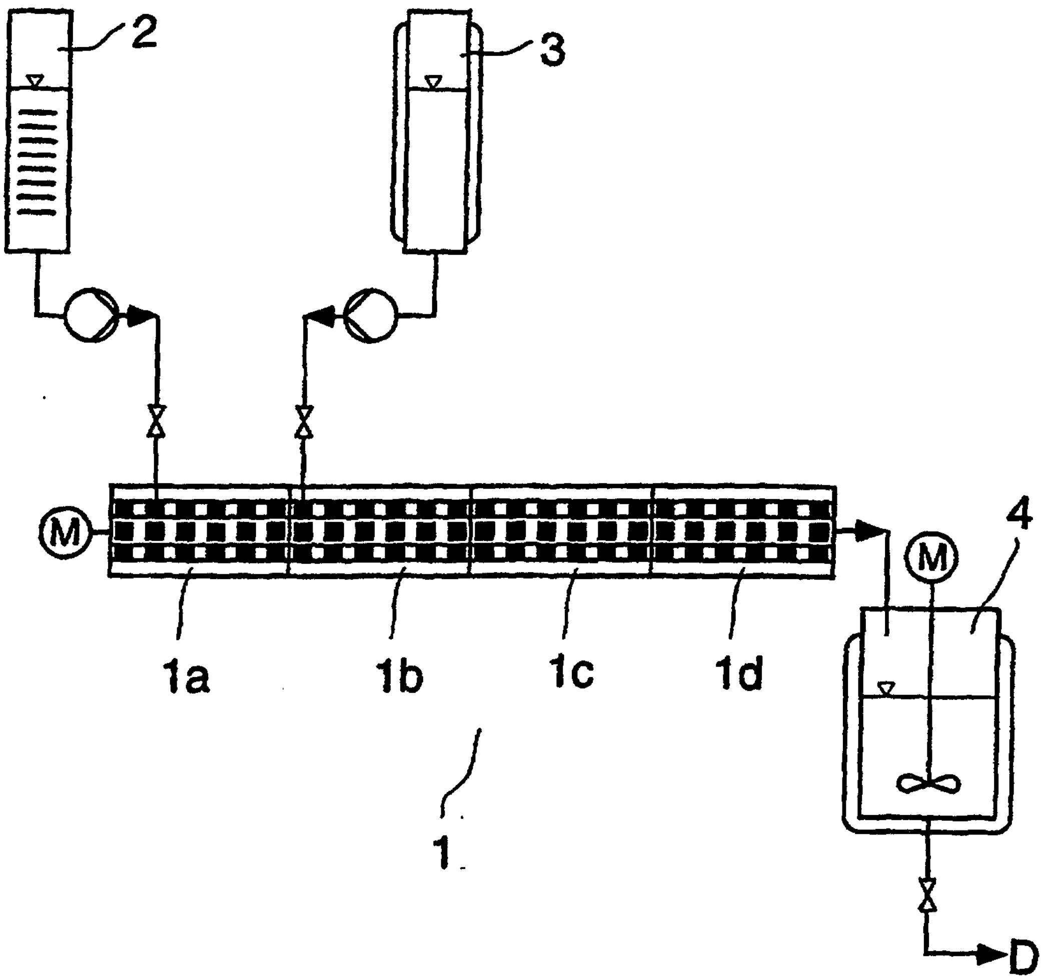
2
3
M
M
4
1a
1b
1c
1d
1
D